# EUROPÄISCHE PATENTSCHRIFT

(11) **EP 1 948 272 B1**
(45) Veröffentlichungstag und Bekanntmachung des Hinweises auf die Patenterteilung: **13.01.2010**
(21) Anmeldenummer: 06806658.8
(22) Anmeldetag: 02.11.2006
(51) Int. Cl.: A61M 5/31, A61M 5/315

(54) **ZYLINDERKOLBENEINHEIT MIT EINER NICHTZYLINDRISCHEN KAMMER**
CYLINDER/PISTON UNIT HAVING A NON-CYLINDRICAL CHAMBER
UNITE CYLINDRE-PISTON AVEC UNE CHAMBRE NON CYLINDRIQUE

(30) Priorität: 14.11.2005 DE 102005054600
(43) Veröffentlichungstag der Anmeldung: 30.07.2008
(73) Patentinhaber: LTS LOHMANN Therapie-Systeme AG, 56626 Andernach (DE)
(72) Erfinder: HOFFMANN, Hans-Rainer, 56566 Neuwied (DE); ASMUSSEN, Bodo, 56170 Bendorf (DE); WORTMANN, Uwe, 35037 Marburg (DE)
(74) Vertreter: Schmidt, Werner
(86) Internationale Anmeldenummer: PCT/EP2006/010506
(87) Internationale Veröffentlichungsnummer: WO 2007/054233

(56) Entgegenhaltungen:
- WO-A-01/60434
- US-A- 3 135 260
- US-B1- 6 213 985

## Beschreibung

Die Erfindung betrifft eine Zylinderkolbeneinheit mit einem Zylinder und einem darin geführten Kolben, wobei der Zylinder und der Kolben eine zumindest zeitweise wirkstoffbefüllbare Kammer umschließen und der Zylinder an seinem vorderen Ende mindestens ein Austrittselement aufweist.

Aus der DE 201 05 183 U1 ist eine Ampulle für ein nadelloses Injektionsgerät bekannt. In der Ampulle befindet sich in einer zylindrischen Kammer ein Arzneimittel, das bei der subkutanen Verabreichung als Flüssigkeitsstrahl mittels eines zylindrischen Kolbens ausgestoßen wird. Der Kolben des handelsüblichen Produkts ist in der zylindrischen Kammer mittel eines Silikongels geschmiert. Bei der Verwendung dieser Ampullen in einem gebräuchlichen Injektionsgerät fällt der Ausstoßdruck über den Kolbenhub erheblich ab. Auch wird mit jeder Arzneimitteldosis das silikonhaltige Kolbenschmiermittel ausgetragen.

Ein weiteres aus dem Stand der Technik bekanntes Injektionsgerät ist im Dokument US 6 213 985 B1 offenbart. Der Oberbegriff des Anspruchs 1 ist auf diesem Dokument basiert.

Der vorliegenden Erfindung liegt daher die Problemstellung zugrunde, eine Zylinderkolbeneinheit zu entwickeln, die bei kleinem Bauvolumen wenige Bauteile benötigt, eine einfache und sichere Handhabung gewährleistet und im befüllten Zustand, gas- und feuchtigkeitsdicht verschlossen, lange lagerbar ist.

Diese Problemstellung wird mit den Merkmalen des Hauptanspruches gelöst. Dabei vergrößert sich im Zylinder der Querschnitt der Kammer oder der Querschnitt der Zylinderinnenwandung zumindest bereichsweise von vorn nach hinten, wobei vorn das Zylinderende mit dem Austrittselement ist. Der Kolben weist zumindest im vorderen Bereich eine elastische Schürze auf, deren vordere Außenkante beim unbelasteten Kolben eine Querschnittsfläche aufspannt, die größer ist als eine Fläche, die von einer Umrisslinie aufgespannt wird.

Mit der Erfindung wird, wie im kennzeichnenden Teil des Hauptanspruchs definiert, eine z.B. in einer Subkutaninjektionsvorrichtung verwendbare Zylinderkolbeneinheit geschaffen, bei der die Zylinderkolbeneinheit einen nach dem technischen Prinzip der Selbsthilfe selbstabdichtenden Kolben aufweist, der u.a. aufgrund seiner Dichtmittelgestaltung schmiermittelfrei im Zylinder sitzt.

Weitere Einzelheiten der Erfindung ergeben sich aus den Unteransprüchen und den nachfolgenden Beschreibungen schematisch dargestellter Ausführungsbeispiele.
- Figur 1:: Zylinderkolbeneinheit mit einem in beiden Endlagen positionierten Kolben;
- Figur 2:: Zylinderkolbeneinheit mit folienverschlossenen Stirnseiten;
- Figur 3:: Draufsicht (50% vergrößert) auf die vordere klebstoffbeschichtete Verschlussfolie;
- Figur 4:: Zylinderkolbeneinheit mit teilweise abgezogener Verschlussfolie;
- Figur 5:: Zylinderkolbeneinheit mit mehreren Düsen;
- Figur 6:: Draufsicht (50% vergrößert) auf den Zylinder zu Figur 5;
- Figur 7:: Teilschnitt durch entleerte Zylinderkolbeneinheit mit Kolben ohne separates Dichtelement;
- Figur 8:: Querschnitte des unbelasteten Kolbens;
- Figur 9:: Druck-Kolbenhub-Diagramm.

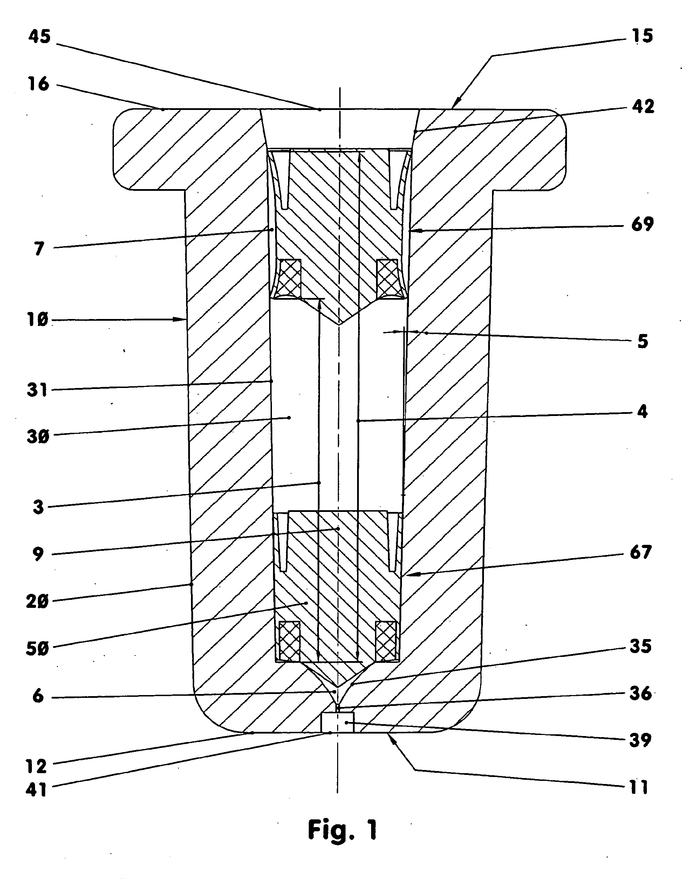

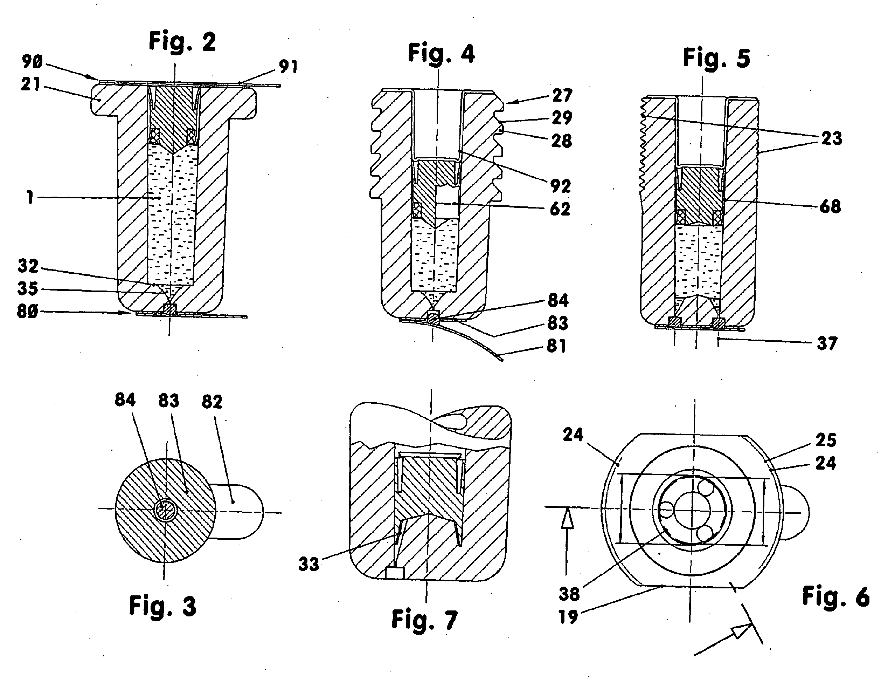

Die Figur 1 zeigt eine Zylinderkolbeneinheit, wie sie beispielsweise in einer Subkutaninjektionsvorrichtung verwendet wird. Sie umfasst einen Zylinder (10) und einen z.B. kolbenstangenfreien Kolben (50). Beide schließen in einer Kammer (30) ein subkutan zu verabreichendes Präparat (1) oder ein flüssiges Trägermaterial, z.B. destilliertes Wasser oder physiologische Kochsalzlösung, ein, vgl. Figuren 2, 4 und 5. Der Kolben (50) ist in Figur 1 zur besseren Veranschaulichung in einer vorderen (67) und einer hinteren Position (69) dargestellt. Die Zylinderkolbeneinheit ist beispielsweise für den einmaligen Gebrauch vorgesehen. Sie dient der Verabreichung eines Arzneinennvolumen von z.B. 0,1 bis 2 ml. Ggf. kann auch ein Arzneinennvolumen von 3 ml realisiert werden. Der hier nur beispielsweise ohne integrierte Injektionsnadel ausgeführte Zylinder (10) hält bei einer Verwendung in einer Subkutaninjektionsvorrichtung einer temporären Druckbelastung von mindestens 300 x 10⁵ Pa stand.

Der Zylinder (10) hat in erster Näherung die Form des Spitzenzylinders einer genormten Einmalspritze. Am vorderen Ende (11) befindet sich ein düsenartiges Austrittselement (36), das in der vorderen - beispielsweise planen - Zylinderstirnseite (12) mit einer z.B. kreisrunden Öffnung (41) einer Freistrahlausnehmung (39) endet. Ggf. kann anstelle des düsenartigen Austrittselements eine in den vorliegenden Figuren nicht dargestellte Injektionsnadel sitzen.

Am hinteren Ende ist ein Adapterflansch (21), ein Rastrippenflansch (27), vgl. Figur 4, ein Gewindeflansch (23), vgl. Figur 5, ein Bajonettflansch oder etwas Vergleichbares angeformt oder befestigt. Auch hier kann die flanschseitige, hintere Zylinderstirnfläche (16) eben und normal zur Zylindermittellinie (9) sein.

Zwischen dem Adapter (21, 23, 27) und der vorderen Stirnfläche (12) befindet sich z.B. eine zylindermantel- oder kegelstumpfmantelförmige Außenkontur (20). Die Form der Außenkontur(20) des Zylinders (10) ist in den meisten Fällen unabhängig von der funktionalen Bauteilbezeichnung "Zylinder (10)". Die Außenkontur (20) kann u.a. eine oder mehrere partielle Abflachungen aufweisen, um ein unbeabsichtigtes seitliches Wegrollen beim Handhaben auf einer ebenen Unterlage zu verhindern.

Der Adapterflansch (21), nach den Figuren 1 und 2, dient wie die anderen Adapterkonturen (23, 27) zur formsteifen, teils höhenvariablen Fixierung an der nicht dargestellten Subkutaninjektionsvorrichtung. Hierbei umgreift ein Bund des Injektorgehäuses oder eine andere Adapterkontur den entsprechenden Flansch des Zylinders (10). Bei einer Injektorvariante mit einem nahezu vollständigen injektorgehäuseseitigen Zylinderumgriff kann ggf. auf einen Adapter verzichtet werden.

Der Außendurchmesser des Adapterflansches ist z.B. mindestens um eine Zylinderwandstärke größer als der Außendurchmesser der benachbarten Außenkontur (20) des Zylinders (10). Die Flanschstärke liegt in der Größenordnung der Zylinderwandstärke. Auch der Flansch kann seitlich zur Behinderung einer Wegrollbewegung eine oder mehrere Abflachungen (19) aufweisen, vgl. Figur 5 und 6. Statt der Abflachung (19) sind auch Kerben, Nuten, Sicken oder Riffelungen denkbar.

In Figur 2 ist ein Zylinder (10) mit einem Rastrippenflansch (27) dargestellt. Die Rastrippen (28) bilden im Querschnitt eine Art Sägezahnprofil mit fünf Zähnen und vier Zahnlücken. Über die nach hinten orientierten zahnabschrägenden 45°-Anfasungen (29) kann der Zylinder (10) in z.B. fünf verschiedenen Rastpositionen in das Injektorgehäuse eingesteckt werden. Ein entsprechender Gehäuseumgriff rastet z.B. elastisch in den entsprechenden Zahnlückenringraum ein.

Das Gewinde (25) des Gewindeflansches (23) nach Figur 5 und 6 bedeckt - bezogen auf den Umfang - ca. 60% der Flanschkontur in zwei einander z.B. gegenüberliegenden Gewindeabschnitten (24).

Der Rastrippenflansch (27) und der Gewindeflansch (23) erstrecken sich in den Ausführungsbeispielen über die hinteren 50% der Zylinderlänge.

Die Innenkontur des Zylinders (10) umfasst - bei einem Zylinder mit nur einem Austrittselement (36) - die Zylinderinnenwandung (31), ggf. mit einer Anfasung (42), einen Zylinderboden (32), einen Ausströmtrichter (35), eine Düsenbohrung (36) und eine Freistrahlausnehmung (39).

Die z.B. glatte Zylinderinnenwandung (31) verjüngt sich nach den Ausführungsbeispielen linear von hinten nach vorn. Sie erstreckt sich zudem nach den Figuren 1, 2, 4 und 5 über den gesamten Kolbenhubbereich (4). Auch sind alle Querschnitte der Zylinderinnenwandung (31) außerhalb des Bereichs des oder der Ausströmtrichter (35) kreisrund. Nur beispielsweise verjüngt sich die Zylinderinnenwandung (31) auf einem Kolbenhub (3) von 18 Millimeter von einem Durchmesser von 7 Millimeter auf 6 Millimeter. Das entspricht einem Verjüngungswinkel von ca. 3,2 Winkelgraden.

Anstelle der dargestellten Spezialfälle können die Querschnitte über den Kolbenhub (39 neben ihrer Fläche auch ihre Form ändern. So könnte die Zylinderinnenwandung an ihrem hinteren Ende z.B. eine ovale Querschnittsform haben, während ein dem vorderen Ende näherliegender Querschnitt eine runde oder polygonartige Form hat. Ferner ist es auch möglich, dass die Querschnittsflächenänderung über den Kolbenhub nicht linear ist. Beispielweise kann zur Verringerung der Kolbenbremswirkung die Verjüngung erst im letzten Drittel des Ausstoßhubs einsetzen. Der Übergang zwischen Abschnitten unterschiedlicher Querschnitte ist in der Regel stetig gestaltet.

Zwischen der Zylinderinnenwandung (31) und der hinteren Stirnfläche (16) kann zur erleichterten Montage des Kolbens (10) eine 15°-Fase (42) vorgesehen sein.

Die Querschnittsverjüngung kann sich ggf. auch nur auf die Kammer (30) beziehen. In diesem Fall befindet sich der in einer hinteren Position (69) angeordnete Kolben (50) auf seiner gesamten Länge in einem Wandungsabschnitt mit z.B. zylindrischer Kontur.

Der Ausströmtrichter (35) verjüngt sich zwischen dem Zylinderboden (32) und der Düsenbohrung (36) zur besseren Strömungsführung nicht linear. Es wird ein stetiger Übergang zwischen dem Ausströmtrichter (35) und der Düsenbohrung (36) angestrebt. Die Düsenbohrung (36), deren Durchmesser z.B. zwischen 0,1 und 0,2 Millimetern liegt, ist zwei- bis viermal so lang wie ihr Durchmesser. An die Düsenbohrung (36) schließt sich eine zylinderkammerartige Freistrahlausnehmung (39) an. Die Ausnehmung (39) hat einen planen Boden, der zudem normal zur Mittellinie der Düsenbohrung (36) orientiert ist. Ihr Durchmesser entspricht dem Acht- bis Sechzehnfachen des Düsenbohrungsdurchmessers, sofern die Ausnehmungstiefe mindestens doppelt so groß ist wie die Düsenbohrungslänge.

U.a. in den Figuren 5 und 6 ist ein Zylinder (10) mit drei Austrittselementen in Form von Düsenbohrungen (36) dargestellt. Die Düsenbohrungen (36) haben Mittellinien (37), die parallel zur Mittellinie (9) sind. Sie sitzen äquidistant verteilt auf einem Lochkreis (38). Letzterer ist nur geringfügig kleiner als der kleinste Kammerdurchmesser im Kolbenhubbereich (4). Zwischen der jeweiligen Düsenbohrung (36) und dem Zylinderboden (32) erstrecken sich schräge Trichter. Zwischen den Trichtern ist der Zylinderboden (32) nach innen gewölbt.

Als Werkstoff für den Zylinder (10) wird ein transparenter, amorpher Thermoplast, z.B. ein(e) Copolymer(e) auf der Basis von Cycloolefinen und Ethylenen oder α-Olefinen (COC) verwendet.

Der im Zylinder (10) geführte Kolben (50) muss die Querschnittsänderung der Zylinderinnenwandung durch eine entsprechende Dichtquerschnittsverringerung ausgleichen. Hierbei soll die Wandungsreibung nur unwesentlich zunehmen dürfen.

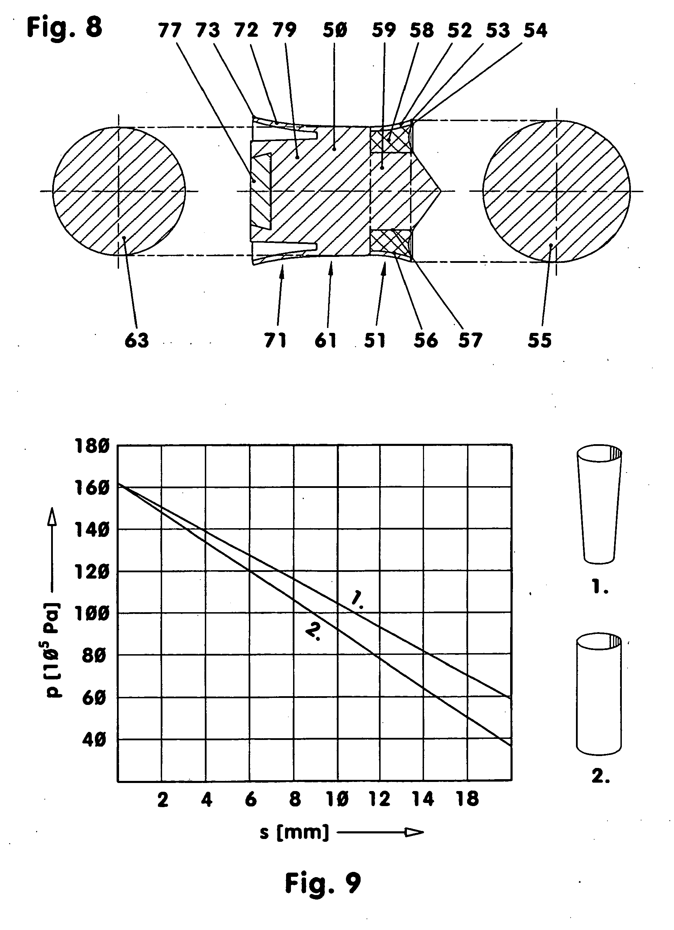

Um dies u.a. zu erreichen hat der in drei Abschnitte (51, 61, 71) aufteilbare Kolben (50) in einem vorderen (51) und hinteren Abschnitt (71) jeweils eine Schürze (52, 72), vgl. Figur 8. Zwischen den Abschnitten (51) und (71) befindet sich der mittige Kolbenabschnitt (61).

Der Mittelabschnitt (61) hat die Form eines Kegelstumpfes. Er passt verformungsfrei in das vordere Ende der Kammer (30). Nach vorn schließt sich mittig ein vorderer Kern (59) an. Um den Kern (59) herum befindet sich die vordere Schürze (52). Zwischen der Schürze (52) und dem Kern (59) liegt nach Figur 8 eine Axialringnut (57). Einen vergleichbaren Aufbau haben auch die hintere Schürze (72) und der hintere Kern (79). Die Schürzen (52, 72), die Kerne (59, 79) und der Mittelabschnitt (61) haben jeweils eine rotationsymmetrische Grundform. Alle erwähnten Teile bzw. Bauteilabschnitte haben deckungsgleiche Mittellinien. Der einzelne Kern (59, 79) steht über die jeweilige Schürze (52, 72) um z.B. wenige Zehntel Millimeter über.

Der vordere Kern (59) hat nach Figur 8, 1, 2 und 4 als Stirnfläche einen geraden, positiven Kegelmantel. Nach den Figuren 5 und 7 ist der Kegelmantel der vorderen Stirnfläche negativ - also nach innen zum Kolbenschwerpunkt hin geformt. Nahezu jede andere rotationssymmetrische Stirnfläche ist denkbar, sofern sie - bei in vorderer Position (67) liegendem Kolben (50) - ein möglichst geringes Restraumvolumen (6) gegenüber dem zumindest bereichsweise anliegenden Zylinderboden (32) zulässt.

Die vordere Schürze (52), die sich über ein Viertel bis ein Drittel der Kolbenlänge erstreckt, ist ein dünnwandiger, sich im unbelasteten Zustand trichterförmig öffnender Ring. Die vordere Außenkante (53) der Schürze (52) umgibt eine Querschnittsfläche (55), die nach Figur 8 größer ist als eine Querschnittsfläche (63), deren Umfang durch eine - am Fuß der Schürze (52) liegende - gedachte Umrisslinie (62) bestimmt wird. Die Umrisslinie (62) ist in einer Teilansicht des Kolbens (10) in Figur 4 strichpunktiert dargestellt.

Während eines Arbeitshubs verändert die Umrisslinie (62) ihre Länge nicht oder kaum, d.h. der von ihr umgebene Querschnitt (63) bleibt im Wesentlichen konstant. Dagegen verkürzt sich die vordere Außenkante (53) - bei linearer Verjüngung der Zylinderinnenwandung (31) - über den gesamten Arbeitshub. In dem vorderen Kolbenhubbereich (4), vgl. Figur 1, ist im Bereich des Dichtelements (58) die vordere Außenkante (53) sogar kürzer als die Umrisslinie (62).

Nach den Figuren 1, 2, 4, 5 und 8 befindet sich in der Axialringnut (57) das Dichtelement (58). Letzteres ist ein separater Dichtungsring oder eine eingeklebte dauerelastische Dichtmasse. Sie verbindet - bei einem in der vorderen Position (67) angekommenen Kolben (50) - bündig die vordere Innenkante (54) der Schürze (52) mit der kernseitig vorderen Stirnfläche. Dies trägt zur Minimierung des Restvolumens (6) in der Kammer (30) bei.

Das Dichtelement (58) kann sich auch innerhalb der Schürze (52) erstrecken, also den vorderen Kern (59) vollständig ersetzen. In beiden Fällen liegt das Dichtelement (58) dichtend an der Schürzeninnenwandung (56) an. Die beim Arbeitshub auftretenden Druckkräfte wirken über das Dichtelement (58) indirekt auf die Schürzeninnenwandung (56).

Des Weiteren ist es möglich, auf das Dichtelement (58) zu verzichten, vgl. Figur 7. Dort ragt die vordere Schürze (52) in eine entsprechende Ringnut (33) hinein.

Nach Figur 8 befindet sich im hinteren Kern (79) des Kolbens (50) eine eingelassene z.B. magnetische oder magnetisierbare Metallplatte (77). Sie deckt beispielsweise 50% der hinteren Querschnittsfläche ab und ist 0,5 bis 1 Millimeter dick. Die Metallplatte (77) erleichtert die Handhabung des Kolbens (50) bei der automatischen Montage der Zylinderkolbeneinheit. Durch die Magnet- und/oder Schwerkraft der Metallplatte (77) lässt sich der Kolben (50) gezielt ausrichten und aufnehmen.

Für den Kolben (50) wird als Werkstoff ein Tetrafluorethylen/Hexafluorpropylen-Copolymer (FEP) verwendet. Dieser hat in Verbindung mit dem zuvor genannten Werkstoff des Zylinders (10) selbstschmierende Eigenschaften, so dass zwischen dem Kolben (50) und dem Zylinder (10) keine separaten Schmiermittel benötigt werden. Als alternative Werkstoffe stehen u.a. Perfluoralkoxy-Copolymer (PFA), Tetrafluorethylen (E TFE) oder Polyvinylidenfluorid (PVDF) zur Auswahl.

Ggf. kann auch eine Werkstoffkombination verwendet werden, bei der der Kernbereich (59, 61, 79) des Kolbens (50) aus einem Material geringerer Elastizität hergestellt wird, während die Schürzen (52, 72) aus einem hochelastischen Werkstoff gefertigt werden.

Nach Figur 1 liegt der Kolben (50) in seiner hinteren Position (69) über die Schürzen (52, 72) federnd an der Zylinderinnenwandung (31) an. Da in diesem Zylinderbereich der Innendurchmesser relativ groß ist, bildet sich zwischen der radialen Kolbenaußenwandung und der Zylinderinnenwandung (31) ein mit Gas oder Luft befülltes Gaspolster (7). Wird nun der Kolben (50) durch ein entsprechendes Antriebsmittel des Subkutaninjektors betätigt, wird durch die hubbedingt enger werdende Zylinderinnenwandung (31) das sich verdichtende Gaspolster (7) entgegen der Kolbenbewegungsrichtung verdrängt. Das Gas entweicht unter Überdruck kontinuierlich zwischen der hinteren Außenkante (73) der Schürze (72) und der Zylinderinnenwandung (31). Hierbei hebt die hintere Schürze (72) im µm-Bereich von der Zylinderinnenwandung ab. Die führende Schürze (72) gleitet nahezu reibungsfrei gasgeschmiert an der Zylinderinnenwandung (31) entlang. Erst in der unteren Kolbenposition (67) ist das Gaspolster (7) nahezu vollständig verdrängt. Demgegenüber liegt die vordere Schürze (52) zumindest über die vordere Außenkante (53) permanent dichtend an der Zylinderinnenwandung (31) an.

Beim Arbeitshub des Kolbens (50) wird die flüssige Zylinderbefüllung (1) über die Düsenbohrung (36) in einem harten Flüssigkeitsstrahl abgegeben. Wird für den Antrieb z.B. eine mechanische, pneumatische oder damit vergleichbare Feder oder ein Federsystem verwendet, so lässt in der Regel die Antriebskraft über den Kolbenhub kontinuierlich nach. Folglich lässt der Druck des Flüssigkeitsstrahls entsprechend nach. Durch das Verringern des Zylinderinnenwandungsquerschnitts über den Kolbenhub (3) wird die wirksame Kolbenfläche zunehmend kleiner. Hierdurch reduziert sich der Druck des.Flüssigkeitsstrahls erheblich weniger als bei einem Zylinder mit zylindrischer Innenwandung vgl. Figur 9.

In Figur 9 sind diese Verhältnisse in einem Druck-Weg-Diagramm dargestellt. Innerhalb des Diagramms ist auf der Abszisse der Druck (p) in Pascal aufgetragen. Auf der Ordinate ist der Kolbenhub (s) in Millimetern angegeben. Die Kurve (1.) zeigt den Druckverlauf bei einer konischen Kammer (30) nach Figur 1, während die Kurve (2.) den Druckverlauf für eine zylindrische Kammer darstellt. Die Kurve (1.) verläuft flacher als die Kurve (2.) Somit steht dem Flüssigkeitsstrahl schon kurz nach dem Strahlbeginn bis zum Aufbrauch der Befüllung (1) ein höherer Druck zur Verfügung, wobei sich die ablesbare Differenz der wegabhängigen Drücke mit zunehmendem Kolbenhub permanent vergrößert.

Bei einer Zylinderkolbeneinheit können die beidseitigen Stirnflächen (12, 16) des Zylinders (10) Öffnungen (41, 45) aufweisen, die mittels gas- und feuchtigkeitsdichten Verschlussmitteln (80, 90) verschlossen sind. Diese Verschlussmittel (80, 90) sind Folien (81, 91) und/oder Beschichtungen (92).

In den Figuren 2, 4 und 5 sind befüllte Zylinderkolbeneinheiten dargestellt. Nach Figur 2 ist die hintere Stirnfläche (16) des Zylinders (10) mit einem Verschlussmittel (90) aus einer abziehbaren gas- und flüssigkeitsdichten Dichtfolie (91) verschlossen. Die silikonierte Dichtfolie (91) ist z.B. eine PET-Folie, eine HTPE-Folie, eine PE-Folie oder eine BOPP-Folie, die auf der Zylinderstirnseite (16) aufgeklebt oder aufgesiegelt wird.

In den Figuren 4 und 5 wird anstelle einer Dichtfolie (91) eine Sprühbeschichtung (92) verwendet. Der aufgesprühte Lack (92) basiert auf einem Zellulosederivat. Er kann auch aus einem vergleichbaren physikalisch unbedenklichen Werkstoff hergestellt sein. Der aufgesprühte Lack (92) legt sich an der hinteren Stirnfläche (16), einem Teil der Zylinderinnenwandung (31) und auf der hinteren Stirnfläche des Kolbens (50) dicht und festhaftend an. Bei der Verwendung dieser so verschlossenen Zylinderkolbeneinheit muss der Lack (92) vor dem Einsetzen in den Injektor nicht entfernt werden. Er wird durch den den Kolben (50) antreibenden Injektorstempel einfach aufgerissen, vgl. Figur 7. Dort ist am Kolben (50) ein anhaftendes Reststück des Lacks (92) sichtbar.

Die Öffnung(en) (41) der vorderen Zylinderstirnfläche (12) ist oder sind mit einer lösbaren Dichtfolie (81) verschlossen, die mindestens zwei verschiedene Klebestoffbereiche umfasst, wobei der erste - um die Öffnung(en) (41) herum angeordnete - Klebstoffbereich - aus einem Haftkleber (83) besteht, der gegenüber der Zylinderstirnfläche (12) ein größere Affinität aufweist als gegenüber der Dichtfolie (81), während der zweite - die Öffnung(en) (41) abdeckende - Klebstoffbereich einen Verschlussklebstoff (84) beinhaltet, der gegenüber der Dichtfolie (81) eine größere Affinität aufweist als gegenüber dem Zylinderwerkstoff.

### Bezugszeichenliste:

- 1: Wirkstoff, Befüllung
- 3: Kolbenhub
- 4: Kolbenhubbereich
- 5: halber Verjüngungswinkel
- 6: Restvolumen
- 7: Gaspolster
- 9: Mittellinie

- 10: Zylinder
- 11: vorderes Ende, Ende mit Austrittselement
- 12: Stirnfläche, vorn

- 15: hinteres Ende
- 16: Stirnfläche, hinten
- 19: Abflachung

- 20: Außenkontur
- 21: Adapterflansch
- 23: Gewindeflansch
- 24: Gewindeabschnitte
- 25: Gewinde
- 27: Rastrippenflansch
- 28: Rastrippen
- 29: Anfasungen

- 30: Kammer
- 31: Zylinderinnenwandung, Innenkontur
- 32: Zylinderboden
- 33: Ringnut

- 35: Ausströmtrichter
- 36: Düsenbohrung, Austrittselement
- 37: Mittellinien der Düsenbohrungen
- 38: Lochkreis, Zylinder auf dem die Mittellinien (37) liegen
- 39: Freistrahlausnehmung

- 41: Öffnung, vorn
- 42: Kammerfase, hinten
- 45: Öffnung, hinten

- 50: Kolben
- 51: Kolbenabschnitt, vorn
- 52: Schürze, vorn, elastisch
- 53: Schürzenaußenkante, vorn
- 54: Schürzeninnenkante, vorn
- 55: Querschnitt zur Außenkante
- 56: Schürzeninnenwandung
- 57: Axialringnut
- 58: Kolbendichtung, Dichtring, Dichtmasse
- 59: Kolbenkern, vorn

- 61: Kolbenabschnitt, mittig, kegelstumpfförmig
- 62: Umrisslinie, gedacht
- 63: Querschnitt zur Umrisslinie (62)

- 67: Kolbenposition, vorn, vordere Endlage
- 68: Kolbenposition, Mitte
- 69: Kolbenposition, hinten

- 71: Kolbenabschnitt, hinten
- 72: Schürze, hinten, elastisch
- 73: Außenkante, hinten
- 77: Platte, magnetisierbar
- 79: Kolbenkern, hinten

- 80: vorderes Verschlussmittel
- 81: Dichtfolie, lösbar
- 82: Abziehlasche
- 83: Haftkleber
- 84: Verschlussklebstoff, Silikonkleber

- 90: hinteres Verschlussmittel
- 91: Siegelfolie, Dichtfolie
- 92: Beschichtung

## Patentansprüche

1. Zylinderkolbeneinheit mit einem Zylinder (10) und einem darin geführten Kolben (50), wobei der Zylinder (10) und der Kolben (50) eine zumindest zeitweise wirkstoffbefüllbare Kammer (30) umschließen und der Zylinder (10) an seinem vorderen Ende (11) mindestens ein Austrittselement (36) aufweist,
- wobei sich der Querschnitt der Kammer (30) oder der Querschnitt der Zylinderinnenwandung (31) zumindest bereichsweise von vorn nach hinten vergrößert,
- wobei der Kolben (50) zumindest in dem - dem Austrittselement (36) zugewandten - vorderen Bereich eine vordere, elastische Schürze (52) aufweist, deren vordere Außenkante (53) beim unbelasteten Kolben (50) eine Querschnittsfläche (55) aufspannt, die größer ist als eine Fläche (63), die von einer Umrisslinie (62) aufgespannt wird, die im Bereich des Übergangs von der Schürze (52) zum schürzentragenden Kolbenabschnitt (61) liegt,
**dadurch gekennzeichnet, dass**
- die Schürze (52) eine Schürzeninnenwandung (56) hat, an der ein elastisches Dichtelement (58) anliegt, und
- das Dichtelement (58) zumindest in der vorderen Kolbenendlage (67) bündig mit der vorderen, inneren Schürzenkante (54) abschließt.

2. Zylinderkolbeneinheit gemäß Anspruch 1, **dadurch gekennzeichnet, dass** die vordere Außenkante (53) der vorderen Schürze (52) bei jeder Kolbenhubposition eine Querschnittsfläche aufspannt, die der Querschnittsfläche der Zylinderinnenwandung (31) entspricht, die durch die Kontaktlinie der Außenkante (53) aufgespannt wird.

3. Zylinderkolbeneinheit gemäß Anspruch 1, **dadurch gekennzeichnet, dass** die Zylinderinnenwandungsquerschnitte im Kolbenhubbereich (4) Kreisflächen sind.

4. Zylinderkolbeneinheit gemäß Anspruch 1, **dadurch gekennzeichnet, dass** sich die Flächen der Zylinderinnenwandungsquerschnitte zumindest über den Kolbenhub (3) linear ändern.

5. Zylinderkolbeneinheit gemäß Anspruch 1, **dadurch gekennzeichnet, dass** sich zwischen der vorderen Schürze (52) und dem Kolbenkern (59) eine sich axial erstreckende Ringnut (57) befindet.

6. Zylinderkolbeneinheit gemäß Anspruch 5, **dadurch gekennzeichnet, dass** das in der Ringnut (57) angeordnete Dichtelement (58) zumindest in der vorderen Kolbenendlage (67) bündig mit der vorderen, inneren Schürzenkante (54) und der Kolbenstirnfläche abschließt.

7. Zylinderkolbeneinheit gemäß Anspruch 1, **dadurch gekennzeichnet, dass** die Länge der vorderen Schürze (52) mindestens 25% der Kolbenlänge beträgt.

8. Zylinderkolbeneinheit gemäß Anspruch 1, **dadurch gekennzeichnet, dass** der hintere Kolbenabschnitt (71) eine hintere Schürze (72) aufweist.

9. Zylinderkolbeneinheit gemäß Anspruch 1 mit mehreren Öffnungen (41) im vorderen Zylinderende (11), **dadurch gekennzeichnet, dass** die Mittellinien (37) der Austrittselemente (36) auf einem Zylinder (38) liegen, dessen Durchmesser um einen Düsenbohrungsdurchmesser kleiner ist als der mittlere Durchmesser des vordersten im Kolbenhubbereich (4) gelegenen Kammerquerschnitts.

## Claims

1. Cylinder/piston unit with a cylinder (10) and with a piston (50) guided therein, the cylinder (10) and the piston (50) enclosing a chamber (30) that can be filled at least temporarily with active substance, and the cylinder (10) having at least one discharge element (36) at its front end (11),
- the cross section of the chamber (30) or the cross section of the inner wall (31) of the cylinder increasing at least in some areas from the front towards the rear,
- the piston (50) comprising, at least in the front area directed towards the discharge element (36), a front, elastic skirt (52) whose front outer edge (53), when the piston (50) is unloaded, covers a cross-sectional surface area (55) that is greater than a surface area (63) covered by a contour line (62) lying in the area of the transition from the skirt (52) to the piston portion (61) supporting the skirt,**characterized in that**
- the skirt (52) has a skirt inner wall (56) on which an elastic sealing element (58) bears, and
- the sealing element (58), at least in the front end position (67) of the piston, is flush with the front, inner skirt edge (54).

2. Cylinder/piston unit according to Claim 1, **characterized in that**, in each stroke position of the piston, the front outer edge (53) of the front skirt (52) covers a cross-sectional surface area corresponding to the cross-sectional surface area of the cylinder inner wall (31) covered by the contact line of the outer edge (53).

3. Cylinder/piston unit according to Claim 1, **characterized in that** the cross sections of the inner wall of the cylinder in the piston stroke area (4) are circular surfaces.

4. Cylinder/piston unit according to Claim 1, **characterized in that** the surfaces of the cross sections of the inner wall of the cylinder change linearly at least over the piston stroke (3).

5. Cylinder/piston unit according to Claim 1, **characterized in that** an axially extending annular groove (57) is located between the front skirt (52) and the piston core (59).

6. Cylinder/piston unit according to Claim 5, **characterized in that**, at least in the front end position (67) of the piston, the sealing element (58) arranged in the annular groove (57) is flush with the front, inner skirt edge (54) and the end face of the piston.

7. Cylinder/piston unit according to Claim 1, **characterized in that** the length of the front skirt (52) is at least 25% of the piston length.

8. Cylinder/piston unit according to Claim 1, **characterized in that** the rear portion (71) of the piston has a rear skirt (72).

9. Cylinder/piston unit according to Claim 1, with several openings (41) in the front end (11) of the cylinder, **characterized in that** the centre lines (37) of the discharge elements (36) lie on a cylinder (38) whose diameter is smaller, by a nozzle bore diameter, than the mean diameter of the frontmost chamber cross section located in the piston stroke area (4).

## Revendications

1. Unité cylindre-piston avec un cylindre (10) et un piston (50) guidé dans celui-ci, dans laquelle le cylindre (10) et le piston (50) délimitent une chambre (30) apte à être remplie au moins temporairement d'une matière active et le cylindre (10) présente au moins un élément de sortie (36) à son extrémité avant (11),
- dans laquelle la section transversale de la chambre (30) ou la section transversale de la paroi intérieure (31) du cylindre augmente au moins par zones de l'avant vers l'arrière,
- dans laquelle le piston (50) présente, au moins dans la région avant - tournée vers l'élément de sortie (36) - une jupe élastique avant (52), dont l'arête extérieure avant (53) embrasse, lorsque le piston (50) est déchargé, une surface de section transversale (55) qui est plus grande qu'une surface (63) qui est embrassée par une ligne périphérique (62) qui est située dans la région de la transition entre la jupe (52) et la partie de piston (61) portant la jupe,
**caractérisée en ce que**
- la jupe (52) présente une paroi intérieure de jupe (56), sur laquelle s'applique un élément d'étanchéité élastique (58), et
- l'élément d'étanchéité (58) se termine, au moins dans la position extrême avant du piston (67), à fleur de l'arête intérieure avant (54) de la jupe.

2. Unité cylindre-piston selon la revendication 1, **caractérisée en ce que** l'arête extérieure avant (53) de la jupe avant (52) embrasse, dans chaque position de la course du piston, une surface de section transversale qui correspond à la surface de section transversale de la paroi intérieure (31) du cylindre, qui est embrassée par la ligne de contact de l'arête extérieure (53).

3. Unité cylindre-piston selon la revendication 1, **caractérisée en ce que** les sections transversales de la paroi intérieure du cylindre sont des surfaces circulaires dans la région de la course de piston (4).

4. Unité cylindre-piston selon la revendication 1, **caractérisée en ce que** les surfaces des sections transversales de la paroi intérieure du cylindre changent linéairement au moins sur la course de piston (3).

5. Unité cylindre-piston selon la revendication 1, **caractérisée en ce qu'**une rainure annulaire (57) s'étendant axialement se trouve entre la jupe avant (52) et le coeur de piston (59).

6. Unité cylindre-piston selon la revendication 5, **caractérisée en ce que** l'élément d'étanchéité (58) disposé dans la rainure annulaire (57) se termine à fleur de l'arête intérieure avant (54) de la jupe et de la face frontale du piston, au moins dans la position extrême avant (67) du piston.

7. Unité cylindre-piston selon la revendication 1, **caractérisée en ce que** la longueur de la jupe avant (52) vaut au moins 25 % de la longueur du piston.

8. Unité cylindre-piston selon la revendication 1, **caractérisée en ce que** la partie arrière (71) du piston présente une jupe arrière (72).

9. Unité cylindre-piston selon la revendication 1 avec plusieurs ouvertures (41) dans l'extrémité avant (11) du cylindre, **caractérisée en ce que** les axes centraux (37) des éléments de sortie (36) sont situés sur un cylindre (38), dont le diamètre autour d'un diamètre de perçage de buse est plus petit que le diamètre moyen de la section transversale de la chambre située le plus en avant dans la région de la course de piston (4).
